# EUROPÄISCHE PATENTSCHRIFT

(11) **EP 2 179 752 B1**
(45) Veröffentlichungstag und Bekanntmachung des Hinweises auf die Patenterteilung: **13.08.2014**
(21) Anmeldenummer: 09170895.8
(22) Anmeldetag: 22.09.2009
(51) Int. Cl.: A61L 31/02, A61L 31/08

(54) **Implantat sowie Verfahren zur Herstellung desselben**
Implant and method for manufacturing same
Implant et son procédé de fabrication

(30) Priorität: 06.10.2008 DE 102008042602
(43) Veröffentlichungstag der Anmeldung: 28.04.2010
(73) Patentinhaber: Biotronik VI Patent AG, 6341 Baar (CH)
(72) Erfinder: Bayer, Ullrich, 18211, Admannshagen-Bargeshagen (DE); Schettler, Jan, 18053, Rostock (DE); Ewert, Günter, 18109 Rostock (DE)
(74) Vertreter: Lindner-Vogt, Karin L.

(56) Entgegenhaltungen:
- EP-A2- 1 941 918
- WO-A1-2006/007972
- WO-A1-2008/045184
- WO-A2-03/083181
- WO-A2-2009/053670

## Beschreibung

Die Erfindung betrifft ein Verfahren zur Herstellung einer intraluminalen Endoprothese, mit einem Körper, wobei der Körper metallisches Material aufweist, sowie intraluminale Endoprothesen erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen, beispielsweise Stents zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Das Grundgitter kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium und/oder Eisen.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemischen Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Aufgrund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus der Druckschrift DE 10 2006 060 501 ist ein Verfahren zur Herstellung einer korrosionshemmenden Beschichtung auf einem Implantat aus einer biokorrodierbaren Magnesiumlegierung sowie ein nach dem Verfahren erhältliches Implantat bekannt, bei dem die Implantatoberfläche nach Bereitstellen des Implantats mit einer wässrigen oder alkoholischen Konversionslösung enthaltend ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PO₃³⁻, PO₄³⁻, HPO₄²⁻, H₂PO₄⁻, OH⁻, BO₃³⁻, B₄O₇³⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻ behandelt wird, wobei eine Konzentration des Ions oder der Ionen jeweils im Bereich von 10⁻² mol/l bis 2 mol/l liegt. Die Behandlung der Implantatoberfläche mit der genannten Konversionslösung bedingt eine anodische Oxidation des Implantats. Sie wird entweder ohne Verwendung einer äußeren Stromquelle (außen stromlos) oder mit einer Stromquelle durchgeführt. Die in dieser Druckschrift beschriebenen Verfahrensbeispiele sowie Elektrolytzusammensetzungen erfüllen jedoch nicht die Erwartungen hinsichtlich Degradationsverhalten und Dilatationsfähigkeit ohne Schichtzerstörung bei der Anwendung für einen Magnesium-Stent.

Aus den Druckschriften US 2008/0086195 A1 und WO 2008/045184 A1 ist eine medizinische Vorrichtung wie ein Katheter oder ein Stent bekannt, bei dem eine polymerfreie Beschichtung mittels eines plasmaelektrolytischen Prozesses (plasma electrolytic deposition PED) aufgebracht wird. Die plasmaelektrolytische Beschichtung wird dafür verwendet, zusätzlich Wirkstoffe in die Beschichtung einzubringen, welche ein Medikament oder ein therapeutisches Mittel beinhalten. Die plasmaelektrolytischen Beschichtung umfasst eine plasmaelektrische Oxidation (plasma electrolytic oxidation PEO), eine Mikro-Lichtbogenoxidation (micro-arc oxidation MAO), eine Plasma-Lichtbogenoxidation (plasma-arc oxidation PAO), eine anodische Funkenoxidation (anodic spark oxidation) und eine elektrolytische Plasmasättigung (plasma electrolytic saturation PES). Die plasmaelektrolytische Beschichtung wird mittels gepulster Wechsel- oder Gleichspannung bei Spannungen zwischen -100 V bis 600 V durchgeführt. Die Stromdichten bewegen sich im Bereich von 0,5 bis 30 A/dm². In den Druckschriften ist als geeigneter Wechselspannungs-Frequenzbereich für die offenbarte Anwendung ein Bereich von 10 bis 100 Hz angegeben. Die plasmaelektrolytische Behandlung beinhaltet die Anwendung von verschiedenen elektrischen Potentialen zwischen der medizinischen Vorrichtung und einer Gegenelektrode, die eine elektrische Entladung (eine Funken- oder Lichtbogenplasmamikro-Entladung) an oder in der Nähe der Oberfläche der medizinischen Vorrichtung erzeugt. Das in den genannten Druckschriften angegebene Verfahren löst somit nicht das oben angegebene Problem.

Folglich besteht die Aufgabe der vorliegenden Erfindung darin, ein Verfahren zur Herstellung einer intraluminalen Endoprothese anzugeben, das eine Degradation des Implantats im gewünschten Zielkorridor ermöglicht. Dabei soll die Degradation zu einem kontrollierbaren Zeitpunkt stattfinden und zusätzlich die Dilatation bzw. Verformung des Implantats ohne nennenswerten Einfluss auf das Degradationsverhalten ermöglichen. Entsprechend besteht die Aufgabe der Erfindung außerdem darin, ein derartiges Implantat zu schaffen.

Die obige Aufgabenstellung wird gelöst durch ein Verfahren umfassend die folgenden Schritte:
a) Bereitstellen des Körpers der intraluminalen Endoprothese,
b) plasmachemische Behandlung mindestens eines Teils der Körperoberfläche in einer wässrigen Lösung mittels Anlegen einer das Plasma erzeugenden elektrischen Wechselspannung an den Körper der intraluminalen Endoprothese, die eine Frequenz von mindestens etwa 1 kHz aufweist, zur Erzeugung einer ersten Schicht auf dem behandelten Teil der Körperoberfläche.

Die vorliegende Erfindung umfasst die Behandlung der Körperoberfläche in einem wässrigen Elektrolytsystem (wässrige Lösung), bei der plasmachemische Effekte direkt an der Oberfläche des Körpers des Implantats entstehen. Das für Mikrosekunden stabile Plasma an der Körperoberfläche erzeugt Reaktionsprodukte, welche zur Ausbildung der ersten Schicht auf der Körperoberfläche führen. Hierbei umfasst der Körper des Implantats mindestens einen Teil des Implantats, vorzugsweise den Hauptteil des Implantats, welcher die mechanische Integrität des Implantats bewirkt.

Der Vorteil des erfindungsgemäßen Verfahrens besteht darin, dass sich durch die plasmachemische Behandlung auf der Körperoberfläche des Implantats Hydroxide und Oxide des metallischen Materials bilden. Diese Schichtzusammensetzung stellt bei Kontakt mit der Körperflüssigkeit einen temporären Korrosionsschutz dar, der eine verzögerte Degradation des metallischen Materials bewirkt. Die im Zuge der verzögerten Degradation des Implantats frei werdenden Partikel werden teilweise von körpereigenen Zellen inkorporiert und/oder weiter abgebaut. Die mittels des erfindungsgemäßen Verfahrens hergestellte degradationshemmende Schicht weist verfahrensbedingt Poren auf, welche zunächst naturgemäß korrosive Schwachstellen bilden, durch die dem Elektrolyt der Kontakt zum metallischen Grundmaterial erleichtert wird. Die vor und nach den Degradationstests durchgeführten oberflächenanalytischen Untersuchungen haben jedoch gezeigt, dass die mit der plasmachemischen Behandlung einhergehende Hydroxidbildung zu einer lokal begrenzten Versiegelung der Porengründe führt.

Die Versiegelung der Porengründe kann beispielsweise anhand des mehrstufigen Verlaufs des pH-Werts des Elektrolyten in einem Korrosionstest (zum Beispiel in künstlichem Plasma) nachgewiesen werden. In den ersten sieben Tagen steigt der pH-Wert erwartungsgemäß von 7,4 auf 8,5 bis 8,7 an. Nach einem nach sieben Tagen durchgeführten Medienwechsel erhöht sich der pH-Wert in überraschender Weise von 7,4 nur noch auf 8,0. Dieses Verhalten zeigt eine geringere chemische Aktivität des metallischen Grundmaterials (insbesondere des Magnesiums) mit dem korrosiven Medium. Dieser Effekt geht einher mit einem Stopp der Querschnittsverjüngung des Materials des Implantat-Körpers, der bereits nach sieben Tagen im metallographischen Querschliff zu verzeichnen ist. Dieser Effekt kann beispielsweise dadurch erklärt werden, dass Hydroxide des metallischen Materials die Poren in der ersten Schicht ausfüllen und damit der Kontakt des Elektrolyten zum metallischen Grundmaterial unterbunden wird. Durch diesen Selbstheilungseffekt wird die Standzeit der Implantate unter Körpermilieubedingungen wesentlich erhöht. Außerdem kann die Degradationsgeschwindigkeit durch Variation der Schichtdicke gesteuert werden. Hierdurch eröffnet sich auch die Möglichkeit, die Degradationsdauer des Implantats an den spezifischen Implantationsort anzupassen (koronar, intercranial, renal etc.)

Das erfindungsgemäße Verfahren zeichnet sich außerdem dadurch aus, dass an den Körper des Implantats eine das Plasma erzeugende Wechselspannung angelegt wird, die eine Frequenz von mindestens 1 kHz aufweist. Diese, verglichen mit dem Stand der Technik hohe Frequenz bewirkt einen hohen energetischen Eintrag in den Körper bzw. die Körperoberfläche des Implantats, so dass die Hydroxide und Oxide des metallischen Materials vergleichsweise tief in die Oberfläche des Implantat-Körpers gelangen. Die erste Schicht haftet somit sehr gut auf der Körperoberfläche.

Aufgrund des anodischen Wirkprinzips der plasmachemischen Beschichtung besteht keine Gefahr der Wasserstoffversprödung. Auch die Gefahr einer mechanischen Beschädigung besteht nicht, da das Aufrauen der Oberfläche nicht mechanisch erfolgt.

Bevorzugt enthält die wässrige Lösung zur plasmachemischen Behandlung Phosphationen, so dass sich neben den Oxiden und Hydroxiden des darunter liegenden metallischen Materials in der ersten Schicht auch Phosphate des Körpermaterials ausbilden, die für eine bessere biologische Verträglichkeit des Implantatmaterials, insbesondere der Beschichtung, sorgen. Dabei entstammen die Phosphationen der Zugabe von Kaliumdihydrogenphosphat und/oder Dikaliumhydrogenphosphat und/oder Kaliumphosphat und/oder Natriumdihydrogenphosphat (Dihydrat) und/oder Heptahydrat und/oder Dodecahydrat in den wässrigen Elektrolyten. Der bevorzugte Konzentrationsbereich liegt dabei zwischen 5 g/l und 200 g/l der zugegebenen Verbindung in der wässrigen Lösung. Eine besonders bevorzugte Konzentration beträgt zwischen 50 und 100 g/l Kaliumdihydrogenphosphat.

In einem weiteren bevorzugten Ausführungsbeispiel weist der Körper mindestens ein zumindest weitestgehend biodegradierbares metallisches Material auf. Die Behandlung des Implantats aus einem biodegradierbaren Material mittels eines plasmachemischen Verfahrens führt dazu, dass bei Kontakt mit der Körperflüssigkeit ein temporärer Korrosionsschutz realisiert wird, der eine verzögerte Degradation des metallischen Materials bewirkt.

Ein weiterer Vorteil des erfindungsgemäßen Verfahrens besteht darin, dass nicht entfernbare Oberflächenkontaminationen des Grundmaterials durch die erste Schicht absorbiert werden und somit den Degradationsprozess nicht zusätzlich beeinflussen. Zudem werden aus der Oberfläche herausragende, teilweise scharfkantige Ausscheidungen des Implantat-Körpers (wie zum Beispiel nicht gelöste Legierungsbestandteile) abgedeckt. Daraus folgt auch eine erhöhte Hämo- bzw. Biokompatibilität.

Außerdem weist die verfahrensbedingt poröse Struktur der ersten Schicht ein hohes plastisches Verformungsvermögen auf. Beispielsweise werden die beim Dilatieren eines Stents entstehenden Mikrorisse durch Energieakkumulation beziehungsweise -dissipation in den zu den Mikrorissen benachbarten Poren gestoppt. Es kommt somit nicht zu einer Delamination der ersten Schicht.

Aufgrund der Existenz der mittels des erfindungsgemäßen Verfahrens hergestellten ersten Schicht vereinfachen sich zudem die Lager- und Transportbedingungen für nach dem erfindungsgemäßen Verfahren hergestellten Implantate, da die Stabilität eines solchen Implantats gegenüber Degradation höher ist als bei unbeschichteten Implantaten.

In einem bevorzugten Ausführungsbeispiel enthält die wässrige Lösung ein Ion oder mehrere Ionen Sr²⁺, die vorzugsweise in einer Konzentration von jeweils 0,05 Mol/l bis 2,0 Mol/l Sr²⁺ in der wässrigen Lösung enthalten sind. Hierdurch werden Strontiumverbindungen in die erste Schicht, d.h. die Oberflächenschicht des Implantats, eingelagert. Dies ist vorteilhaft, da insbesondere Strontiumcarbonat kaum wasserlöslich ist und somit einen die Degradation besonders hemmenden Schichtbestandteil in der Oberflächenschicht des Implantats bildet. Zudem kann das in der Beschichtung enthaltene Strontiumcarbonat insbesondere bei cranialen Anwendungen eine arzneimittelähnliche Wirkung gegen Zerebralsklerose entfalten.

In einem weiteren, besonders bevorzugten Ausführungsbeispiel der vorliegenden Erfindung wird vor der plasmachemischen Behandlung auf mindestens ein Teil der Körperoberfläche eine zweite Schicht aufgebracht, welche mindestens ein Element, vorzugsweise zwei Elemente, der Gruppe bestehend aus Tantal, Niob, Zink, Eisen und Aluminium, insbesondere nanokristallines Aluminium, aufweist. Eine solche Beschichtung wird insbesondere für Implantat-Körper aus Materialien wie Edelstahl 316L, Nickel-Titan-Legierungen sowie Kobalt-Chrom-Legierungen (z. B. L605) verwendet.

Die genannten Materialien weisen den Nachteil auf, dass diese Nickelionen freisetzen, deren Einfluss auf den Behandelten nicht unkritisch betrachtet wird. Gleiches gilt auch für das Herauslösen von Vanadiumionen aus der in der Orthopädie weit verbreiteten Legierung TiAl6V4. Derartige Freisetzungseffekte werden auch dann beobachtet, wenn auf diesen Materialien Diffusionsbarrieren aus arteigenen oder artfremden Materialien aufgebracht werden.

Gegenwärtig weit verbreitete Oberflächenbehandlungsverfahren für derartige Implantate beinhalten das Elektropolieren. Dieses erzeugt bzw. verstärkt die Wirkung von Passivierungsschichten, welche beispielsweise aus Chromoxiden bzw. Titanoxiden bestehen können. Die durch das Elektropolieren sehr glatten Oberflächen des Implantatkörpers erweisen sich jedoch als nachteilig z.B. für die Haftung anschließend aufzubringender polymerer Deckschichten, die als Reservoir für Wirkstoffe dienen sollen. Zur Verbesserung der Adhäsion wäre eine eher aufgeraute Oberfläche wünschenswert, welche beispielsweise mittels chemischer Ätzverfahren (z.B. Beizen in Säurelösungen) erzeugt werden könnte. Durch ein solches Ätzverfahren wird die Passivierungsschicht jedoch nachteilig zerstört und die Gefahr einer Wasserstoffversprödung erhöht.

Ein weiterer Aspekt bei der Herstellung von Endoprothesen, der eine wünschenswerte Ausgestaltung beinhaltet, umfasst die Röntgensichtbarkeit der Implantate. Üblicherweise wird diese durch konstruktive Änderungen beispielsweise durch den Einbau von Röntgenmarkern und/oder das großflächige Aufbringen schwerer, besser röntgensichtbarer Metallschichten, z.B. Gold, gelöst. Eine solche Schicht führt jedoch zu einem geringen Korrosionswiderstand des Werkstoffverbundes, da sich Lokalelemente bilden. Zudem weist eine Goldbeschichtung eine zu geringe Härte auf.

Die erfindungsgemäße Lösung nach dem bevorzugten Ausführungsbeispiel besteht nun darin, dass vor der plasmachemischen Behandlung auf die Körperoberfläche, insbesondere bei Materialien des Implantatkörpers, welche Nickel und/oder Vanadium enthalten, eine zweite Schicht aufgebracht wird, welche mindestens ein Element der Gruppe bestehend aus Tantal, Niob, Zink, Eisen und Aluminium, insbesondere nanokristallines Aluminium, aufweist. Ein solcher Schichtverbund aus einer zweiten Schicht und einer darüber liegenden, mittels plasmachemischer Behandlung hergestellten ersten Schicht hat den Vorteil, dass durch das metallische Tantal, Niob, Zink, Eisen, und/oder Aluminium, insbesondere nanokristallines Aluminium, eine verstärkte Diffusionsbarriere geschaffen wird, welche die Biokompatibilität für nicht biokompatible Metallionen (Ni, V) gewährleistet.

Als vorteilhaft hat sich weiter erwiesen, dass durch die mittels der ersten Schicht erzeugten Oberflächenrauhigkeit eine erhöhte Adhäsionswirkung für nachfolgende, degradierbare oder nichtdegradierbare polymere oder anorganische Deckschichten bewirkt wird, welche beispielsweise als wirkstofffreisetzende Trägermedien (pharmazeutisch aktive Substanz) dienen können.

Außerdem kann die Porenstruktur der ersten Schicht als Stoffreservoir für pharmazeutisch aktive Substanzen fungieren, welche als Nano- oder Mikropartikel eingelagert werden können und als Gleitmittel zur Verringerung des Reibungskoeffizienten im Katheter, knochenwachstumsfördernde Substanzen wie Kalziumphosphate, temporär wirkende Kontrastmittel oder zellwachstumshemmende radioaktive Substanzen dienen können.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel, oder Sirolimus, bestehen.

Durch die tantal- und/oder niobhaltige Beschichtung wird außerdem eine verbesserte Röntgensichtbarkeit des Implantats erreicht. Unter Umständen kann hierdurch der Einsatz eines speziellen Röntgenmarkers vermieden werden.

Durch den hohen Energieeintrag während der plasmachemischen Behandlung wird außerdem gewährleistet, dass die zweite Schicht sehr gut auf dem Körper des Implantats haftet, da ein Großteil der Lichtbogen-Entladungen so tief in die Körper-Oberfläche hineinwirkt, dass die zweite Schicht an der Grenzfläche zur Körper-Oberfläche fest verbunden wird.

Weitere Vorteile der Kombination aus erster und zweiter Schicht sind:
- erhöhte Korrosionsstabilität durch Verzicht auf den Kontakt mit lokalelementbildenden Schichtwerkstoffen,
- erhöhte Oberflächenhärte, welche zu einer verbesserten mechanischen Handhabung und verringerter Kratzempfindlichkeit führt,
- erhöhte Korrosionsstabilität durch erhöhten Abrasionswiderstand des Implantats in Kontakt mit weiteren Implantaten (z.B. Stent-unterstütztes Coiling von intercranialen Aneurysmen) oder in Kontakt mit Knochen, Gefäßen, Geweben oder anderen Organen und Organbestandteilen des mit dem Implantat Behandelten (z.B. bei der Implantation von Osteosynthesen-Implantaten, beispielsweise beim Einschlagen von Nägeln, Schraubenkontakt/Platte),
- verbesserte Benetzbarkeit mit Polymerlösungen, so dass sich beim Tauchen oder beim Sprühen in oder mit Polymerlösungen technologische Vorteile ergeben, oder
- Erleichterung einer nachträglichen Versiegelung von Implantat-Oberflächen mit aus der Dampfphase abscheidbaren Polymeren.

Bevorzugt wird die zweite Schicht mittels einer ionischen Flüssigkeit, Sputtern, Hochratenzerstäuben und/oder Bedampfen aufgebracht. Der Vorteil des Aufbringens der zweiten Schicht mittels einer ionischen Flüssigkeit besteht darin, dass beim Aufbringen der Schicht, insbesondere bei Verwendung eines binären Systems, Mikrolegierungseffekte zwischen dem Material des Implantatkörpers und dem aufgebrachten Material stattfinden. Durch die Mikrolegierung wird eine mehrere Nanometer bis wenige Mikrometer dicke Schicht 20 ausgebildet, welche eine Legierung aus den in der ionischen Flüssigkeit enthaltenen Metallen und dem Material des Implantatkörpers darstellt, die unter den üblichen schmelzmetallurgischen Bedingungen (z.B. aufgrund fehlender oder zu geringer gegenseitiger Löslichkeit) nicht erzeugbar wäre. Die Schichtkombinationen können sowohl auf einem unbehandelten Implantat als auch auf einer vorher elektrolytisch polierten Implantatoberfläche abgeschieden werden. Eine solche zweite Schicht verhindert die Elution zytotoxischer Metallionen aus dem Implantatkörper, erhöht den Korrosionswiderstand der Oberfläche auch im stark verformten Zustand und ermöglicht so die Behandlung hochreaktiver Implantat-Metalloberflächen (z.B. aus einer Magnesiumlegierung), auf denen sich bei Luftkontakt oder bei der Elektropolitur in Kontakt mit temporären, kurzlebigen Verbindungen sofort eine Oxidschicht oder andere unerwünschte Oberflächen bilden würden. Ein weiterer Vorteil der Beschichtung mittels einer ionischen Flüssigkeit besteht darin, dass die so hergestellte Oberfläche einen erhöhten Korrosionswiderstand auch im stark verformten Zustand gewährleistet. Die Oberfläche der zweiten Schicht ist mikrostrukturiert und verbessert die Haftung einer darüber liegenden Schicht. Weiterhin ist von Vorteil, dass eine so hergestellte zweite Schicht die Diffusion von Ni-, Co-, V- und Cr-lonen aus dem Material des Implantatkörpers zum Zellgewebe langzeitstabil unterbindet.

Vorzugsweise wird die Beschichtung mittels einer ionischen Flüssigkeit durchgeführt, welche die äußerst reaktionsstabile Pyrrolidinium-lonen enthält. Eine solche ionische Flüssigkeit ist ausreichend stabil, um insbesondere eine Abscheidung von nanokristallinem Aluminium zu ermöglichen.

In einem weiteren Ausführungsbeispiel wird das Implantat nach der plasmachemischen Beschichtung in einem Lösungsmittel, vorzugsweise destilliertem H₂O, gespült und danach vorzugsweise bei einer Temperatur von mindestens etwa 80°C, besonders bevorzugt mindestens etwa 100°C getrocknet, wobei die Trocknung vorzugsweise in einem Umluftofen durchgeführt wird.

In einem bevorzugten Ausführungsbeispiel des erfindungsgemäßen Verfahrens sind in der wässrigen Lösung, die für die plasmachemische Behandlung verwendet wird, zusätzlich ein oder mehrere Ionen ausgewählt aus der Gruppe der Carbonate, Hydroxide und Silikate enthalten.

Um den pH-Wert des Elektrolyten (wässrigen Lösung) konstant zu halten, ist bevorzugt in der wässrigen Lösung ein Puffer, vorzugsweise Kaliumdihydrogenphosphat und/oder Natriumdihydrogenphosphat enthalten.

In einem weiteren bevorzugten Ausführungsbeispiel wird der Implantat-Körper vor der plasmachemischen Behandlung elektrochemisch behandelt, vorzugsweise elektrochemisch poliert. Hierdurch werden Verunreinigungen auf der Oberfläche des Implantat-Körpers entfernt, so dass die plasmachemische Behandlung an einer definierten Oberfläche erfolgt. Dabei kann die Elektropolitur (muss aber nicht zwangsläufig) vor der Aufbringung der zweiten Schicht erfolgen. Sie kann auch nach dem Aufbringen der zweiten Schicht und vor der plasmachemischen Behandlung erfolgen. Wichtig ist die Freiheit von Oberflächenkontaminationen, die sonst zu einer schlechten Haftung der plasmachemisch aufgebrachten Beschichtung führen würde. Das Elektropolieren führt zu kontaminationsfreien Deckschichten aufgrund großer Materialabtragseffekte (Tiefenwirkung).

Bevorzugt erfolgt die plasmachemische Behandlung des Implantat-Körpers dadurch, dass an den Körper eine gepulste, vorzugsweise positive, Spannung angelegt wird, deren Amplitude im überwiegenden Zeitraum der Behandlung mindestens etwa 90 Volt übersteigt, besonders bevorzugt mindestens etwa 100 Volt übersteigt und welche vorzugsweise im Verlauf der Behandlung ansteigt. Durch diesen hohen gepulsten Spannungen mit einer Pulslänge von vorzugsweise maximal etwa 50 Mikrosekunden, besonders bevorzugt etwa 5 Mikrosekunden, werden an der Oberfläche des Implantat-Körpers über Mikrosekunden Plasmen erzeugt, welche zur Reaktion des metallischen Materials des Implantatkörpers mit dem Elektrolyten führen. Zwischen den Spannungspulsen folgt eine Ruhephase von vorzugsweise ca. 100 Mikrosekunden.

Bevorzugt wird der plasmachemische Prozess mit einer Stromdichte von mindestens etwa 8 mA/cm² vorzugsweise mindestens etwa 10 mA/cm² durchgeführt.

In einem weiteren, besonders bevorzugten Ausführungsbeispiel wird nach der plasmachemischen Behandlung eine Nachbehandlung des Körpers des Implantats in einer wässrigen, stark basischen Natriumhydroxidlösung zur Erzeugung einer dritten Schicht durchgeführt. Der pH- Bereich liegt dabei vorzugsweise - je nach NaOH-Konzentration - zwischen etwa 11 und etwa 13,8. Die Temperatur der Nachbehandlungslösung liegt bevorzugt zwischen Raumtemperatur und 70°C. Die bevorzugte Verweildauer des Implantatkörpers in der Natriumhydroxidlösung beträgt zwischen 5 s und 180 s.

Durch die Nachbehandlung erfolgt eine Versiegelung der aus der Sicht der Korrosionsbeständigkeit bzw. Durchlässigkeit kritischen Porengründe der durch die plasmachemische Beschichtung erzeugten ersten Schicht, so dass die dritte Schicht im Wesentlichen in den Porengründen vorliegt. Durch die Nachbehandlung werden lediglich die Porengründe und nicht die kompletten Poren verschlossen, so dass der positive Effekt der durch die plasmachemische Behandlung aufgerauten Oberfläche und der damit verbundnen hohen Adhäsion zu polymeren Deckschichten erhalten bleibt. Hierdurch wird eine erhöhte Korrosionsbeständigkeit des Implantats erzielt, die zu einer Degradation und Integritätsverlust in einem für viele Behandlungen interessanten Zeitfenster von 3 bis 12 Monaten führt.

Der bei diesem bevorzugten Ausführungsbeispiel erfindungsgemäß erzielte, auf der Bildung von Hydroxiden (z.B. von Magnesiumhydroxiden bei einem Körper mit Magnesium) beruhende Versiegelungseffekt des Porengrundes bewirkt zudem einen sehr vorteilhaften Rissstopmechanismus. Dieser entsteht aufgrund des Unterschieds der mechanischen Konsistenz des Materials der porösen Deckschicht einerseits und dem Material des versiegelten Porengrundes andererseits. Er beruht auf der hohen Aufnahmefähigkeit der Pore für die Rissenergie.

Mikrorisse entstehen beispielsweise im Moment der Dilatation von Stents oder anderen Implantaten, wenn diese in mikroskopischen Bereichen über ihr Plastifizierungsvermögen hinaus beansprucht werden und führen zu einer unkontrollierten und deshalb unerwünschten Degradation des Grundmaterials des Implantatkörpers. Die bei dem bevorzugten Ausführungsbeispiel in der plasmachemisch erzeugten, aus größtenteils röntgenamorphen Oxiden, Hydroxiden und/oder Phosphaten des Grundmaterials bestehenden Schicht auftretenden Risse werden in den Porengründen aufgefangen. Dies beruht einerseits auf der ruhenden und damit Rissenergie akkumulierenden Geometrie der Pore an sich und andererseits auf der in dem Porengrund angeordneten und im Vergleich zur übrigen Schichtmatrix zähen Hydroxid (z.B. Magnesiumhydroxid). Die Hydroxidschicht auf dem Porengrund wird durch die erfindungsgemäße Nachbehandlung vorteilhaft verstärkt, so dass der beschriebene Rissstoppmechanismus besonders wirksam funktioniert. Hierdurch wird eine hohe Schadenstoleranz bei mechanischer Beanspruchung erzielt, so dass die Degradation gleichmäßiger stattfindet. Vorzugsweise erfolgt die Nachbehandlung unter Beaufschlagung von Ultraschall und/oder Einblasen von Argon und/oder Stickstoff. Die im Wesentlichen auf dem Grund der Poren der ersten Schicht entstehende dritte Schicht weist eine Dicke von einigen 10 nm auf.

Ein weiterer Vorteil der erfindungsgemäßen Kombination der ersten und der dritten Schicht ist, dass keine Blasenbildung und damit kein Lumenverlust bei fortgeschrittener Degradation aufgrund von Semipermeabilität des Schichtverbunds stattfindet bzw. die Blasenbildung reduziert ist.

In einem weiteren bevorzugten Ausführungsbeispiel wird nach der plasmachemischen Behandlung oder nach der oben beschriebenen Nachbehandlung auf die erste Schicht und/oder die dritte Schicht eine vierte, vorzugsweise ein Polymer enthaltende Schicht, besonders bevorzugt zumindest überwiegend aus Parylene bestehend, aufgebracht. Bevorzugte Schichtdicken der Parylene-Schicht liegen zwischen etwa 0,5 und etwa 10 µm. Durch eine derartige Schichtkombination kann die Degradationszeit des Implantats nochmals wesentlich erhöht werden. Vorteilhaft wirkt sich auch die hohe Spaltgängigkeit von Parylene aus, so dass auch eine tiefgehende Penetration der Poren der ersten und/oder dritten Schicht bis hin zu den Porengründen durch Parylene erfolgt. Die für Parylene, insbesondere Parylene N charakteristischen Permeationseigenschaften für Wasser, chloridhaltige Lösungen und Wasserstoff sorgen in Verbindung mit der darunter liegenden plasmachemisch erzeugten und ggf. an den Porengründen versiegelten Oberfläche für ein steuerbares Degradationsverhalten des Implantats. Dieses zeichnet sich auch durch einen über den Implantatquerschnitt uniformen langsamen Korrosionsfortschritt aus. Zudem leistet die Parylene-Schicht einen zusätzlichen Beitrag zur Vermeidung bzw. Behinderung des Rissfortschritts bei mechanischer Belastung und verhindert partielle Schichtablösungen.

Hierbei ist Parylene die Bezeichnung für vollständig lineare, teilkristalline, unvernetzt aromatische Polymere. Die verschiedenen Polymere besitzen unterschiedliche Eigenschaften und lassen sich in vier Grundtypen einteilen, nämlich Parylene C, Parylene D, Parylene N und Parylene F. Für die Anwendung als vierte Schicht im erfindungsgemäßen Schichtverbund wird vorzugsweise Parylene N verwendet.

Die unter der vierten Schicht angeordnete erste Schicht und/oder dritte Schicht führt/führen aufgrund ihrer porösen Struktur zu einem hohen Haftungsvermögen der vierten Schicht, so dass eine sonst vorzuschaltende Primerbehandlung überflüssig wird.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf.

Vorzugsweise weist die mittels der plasmachemischen Behandlung erzeugte erste Schicht eine Dicke von etwa 1 bis 20 µm, vorzugsweise etwa 1 bis 8 µm auf. Eine Schicht mit einer Dicke von etwa 2 bis 5 µm ist aufgrund des höheren Plastifizierungsvermögens zu favorisieren.

Vorzugsweise weist die zweite Schicht eine Schichtdicke zwischen etwa 0,5 µm und etwa 15 µm auf. Dieser bevorzugte Schichtdickenbereich ist von Vorteil, da einerseits eine genügend große Diffusionsbarriere zwischen dem angreifenden korrosiven Medium (Blut bzw. Plasma) und andererseits keine zu große Beeinträchtigung des Umformvermögens (wichtig bei der Stentdilatation) zu verzeichnen ist. So käme es im Falle zu großer Schichtdicken zum bereichsweisen Abplatzen der Schicht beim Crimpen oder Dilatieren, so dass darunter liegende Schichten und das metallische Grundmaterial in direkten Kontakt mit den korrosiven Medien treten würden. Eine beschleunigte Korrosion wäre die Folge.

Oben wurde bereits beschrieben, dass die erste Schicht Poren aufweist, wobei vorzugsweise, insbesondere bei einer vorstehend beschriebenen Behandlung des Implantats in einer Natriumhydroxidlösung, auf dem Porengrund als dritte Schicht ein Hydroxid des metallischen Materials oder der metallischen Materialien des Implantat-Körpers gebildet ist.

Bevorzugt enthält die erste Schicht mindestens eine Verbindung ausgewählt aus der Gruppe Phosphate, Hydroxide und Oxide des biodegradierbaren Materials oder der biodegradierbaren Materialien, Strontiumkarbonat, Strontiumphosphat.

Die erfindungsgemäßen Verfahren werden nachfolgend anhand von Beispielen und Figuren erläutert. Dabei bilden alle abgebildeten und/oder beschriebenen Merkmale den Gegenstand der Erfindung, unabhängig von ihrer Zusammenfassung in den Ansprüchen oder deren Rückbeziehung.

Es zeigen schematisch:
- Figur 1: einen Querschnitt der Schichtstruktur eines Ausführungsbeispiels des erfindungsgemäßen Implantats hergestellt gemäß Beispiel 2 und
- Figur 2: einen Querschnitt durch einen Ausschnitt der Schichtstruktur eines weiteren Ausführungsbeispiel des erfindungsgemäßen Implantats hergestellt gemäß Beispiel 8, insbesondere ein Querschnitt durch eine Pore der ersten Schicht.

### Beispiel 1:

Zunächst wird ein Stent mit einem Körper bestehend aus einer Magnesiumlegierung, vorzugsweise WE43 (93 Gew.% Magnesium, 4 Gew.% Yttrium (W) und 3 Gew.% Seltenerdmetalle (E) außer Yttrium) bereitgestellt.

Anschließend wird die wässrige Lösung (Elektrolyt) dargestellt. Hierzu wird zunächst in ein Becherglas 500 ml destilliertes H₂O gefüllt. Danach werden in der nachfolgend angegebenen Reihenfolge die Komponenten der wässrigen Lösung hinzugegeben. Hierbei ist darauf zu achten, dass die nächstfolgende Komponente erst nach völliger Auflösung der vorangegangenen Komponenten zugegeben werden darf. Der Elektrolyt ist bei der Zugabe ständig zu rühren, was beispielsweise mit einem Magnetrührer (500 min⁻¹) realisiert werden kann. Die Zugabe der Komponenten sollte außerdem langsam erfolgen, um eine übermäßige Wärmeentwicklung zu vermeiden. Vorzugsweise kann die Elektrolytsynthese in einem doppelwandigen, kühlbaren Behälter erfolgen. Folgende Komponenten können zugegeben werden (alle nachfolgenden %-Angaben bedeuten Gew.%)
- 1.: 100 ml/l Ethylendiamin-Lösung (99%),
100 g/l Kaliumdihydrogenphosphat,
90 g/l Strontiumhydroxid-Octahydrat und
40 ml/l wässrige Ammoniumhydroxidlösung (25%)
oder
- 2.: 50 ml/l Ethylendiamin-Lösung (99%),
50 g/l Kaliumdihydrogenphosphat,
90 g/l Strontiumnitrat und
20 ml/l wässrige Ammoniumhydroxidlösung (25%) oder 25 g/l Natriumhydroxid.

Nach der Fertigstellung einer der beiden mit 1. oder 2. bezeichneten Ansätze folgt der plasmachemische Beschichtungsprozess.

Vor dem plasmachemischen Beschichtungsprozess kann ggf., je nach Kontaminationszustand des Implantat-Körpers, ein mehrstufiges Entfetten in tensidhaltigen Lösungen und anschließendes Spülen in destilliertem H₂O erfolgen.

Vor Beginn des plasmachemischen Beschichtungsprozesses wird der Körper des Implantats elektrisch kontaktsicher mittels eines Titan- oder eines Aluminiumdrahts mit der Anode von Elektroden verbunden. Die Gegenelektrode (Kathode) besteht aus einem rost- und säurebeständigen Stahl. Anode und Kathode der Elektroden werden mit einer Spannungsquelle verbunden, welche in der Lage ist, eine gepulste Spannung abzugeben. Die Stromdichte beträgt ca. 10 mA/cm² bei einer Frequenz von mindestens 1 kHz.

Nach dem Eintauchen des Implantat-Körpers in die wässrige Lösung wird eine ständig steigende, gepulste Badspannung angelegt. Beim Erreichen des für die Behandlung von Magnesiumlegierungen in der Elektrolytzusammensetzung charakteristischen Badspannungsbereichs von mehr als 100 V kommt es zu plasmachemischen Oberflächeneffekten. Diese erzeugen Oberflächenschichten, die sowohl aus Oxiden, Hydroxiden und Phosphaten des metallischen Grundmaterials des Implantat-Körpers als auch aus weiteren Verbindungen bestehen, die sich aus Elementen des Elektrolyten rekrutieren. Insbesondere wird aufgrund der Anwesenheit des Sr²⁺-lons auch Strontiumkarbonat gebildet, das sich aus dem Kohlenstoff des Ethylendiamin, dem Sauerstoff des wässrigen Elektrolyten und dem Strontium des Strontiumhydroxides bzw. Strontiumnitrats zusammensetzt. Außerdem wird Strontiumphosphat gebildet, das ein plasmachemisches Umwandlungsprodukt des Kaliumdihydrogenphosphates und des Wassers ist.

Nach dem Erreichen der Beschichtungsendspannung von ca. 250 V sinkt die vorher eingestellte Stromdichte von ca. 10 mA/cm² auf ca. 6 mA/cm² ab. Wird dieser Wert erreicht, so wird die Stromzufuhr unterbrochen und der plasmachemische Prozess beendet. Die Schichtdicke der so erzeugten ersten Schicht beträgt vorzugsweise etwa 1 µm bis etwa 20 µm, besonders bevorzugt zwischen 2 µm und 8 µm, und hängt wesentlich von der verwendeten Badspannung ab.

Anschließend erfolgen ein mehrstufiges Spülen des Implantatkörpers in destilliertem Wasser, eine Trennung des Implantats vom Kontaktdraht und eine Trocknung des Körpers des Implantats in einem Umluftofen bei ca. 100°C.

Mittels des beschriebenen plasmachemischen Beschichtungsprozesses können Implantate hergestellt werden, welche in einem gewünschten Zeitfenster degradieren. Überraschender Weise lässt sich hierbei die Degradation durch eine Schicht steuern, welche porös ausgebildet ist.

### Beispiel 2:

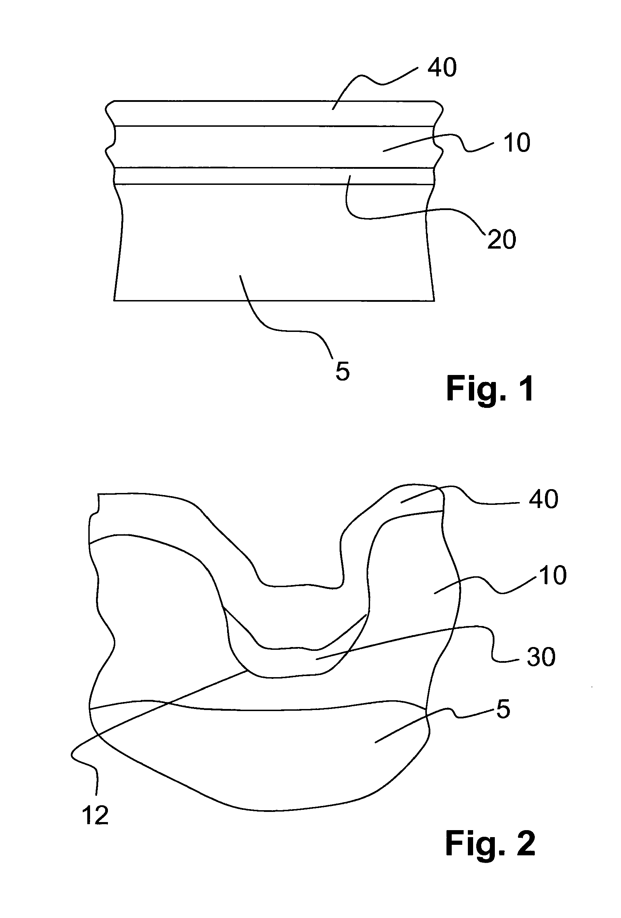

Das Beispiel beschreibt die Beschichtung von nicht resorbierbaren Implantaten aus Nitinol, Edelstahl oder Cobalt-Chromlegierungen oder abbaubaren Werkstoffen mit einer zweiten Schicht 20 aus Metallen enthaltend mindestens ein Element der Gruppe Tantal, Niob, Zink, Eisen und Aluminium. Dabei wird das Implantat zunächst mit bekannten Technologien wie Laserschneiden, Elektropolieren usw. hergestellt, so dass ein Körper 5 (siehe Figur 1) vorliegt. Danach erfolgt eine Beschichtung der Oberfläche des Körpers 5 mit Tantal, Niob, Zink, Eisen, Aluminium oder einer diese Metalle enthaltenden Legierung. Die zum Einsatz kommenden Beschichtungstechnologien können dabei ionische Flüssigkeiten, Sputtern, Hochratenzerstäuben oder auch Bedampfen sein.

Vorzugsweise erfolgt die Herstellung der zweiten Schicht 20 mit Tantal auf einem Nitinol-Implantat mittels einer ionischen Flüssigkeit 1-Butyl-1-methylpyrrolidiniumbis(trifluoromethylsulfonyl)amid aus TaF₅. Hieraus entsteht eine 0,5 µm bis 1 µm dicke Tantal-Schicht 20, welche durch eine anodische Voroxidation von Nitinol in der ionischen Flüssigkeit derart fest haftet, dass sie mechanisch nur sehr schwer zu entfernen ist.

In einem weiteren Ausführungsbeispiel kann ein Magnesium-Implantat, dessen Körper 5 aus einer Magnesium-Legierung besteht, in der oben angegebenen ionischen Flüssigkeit aus AlCl₃ mit nanokristallinem Aluminium beschichtet werden, so dass eine zweite Schicht 20 entsteht, die nanokristallines Aluminium enthält.

Die sich bildende zweite Schicht 20 weist eine Dicke im Bereich von einigen Mikrometern auf. Der vorzugsweise einzustellende Schichtdickenbereich liegt dabei zwischen0,5 und 15 µm. Besonders anzustreben sind Beschichtungsparameter, die zu einer gleichmäßigen Schichtdickenverteilung der zweiten Schicht 20 auch auf der Innenseite bzw. an schwer zugänglichen Spalten und Hinterschneidungen des Implantats führen.

Nach der Herstellung der zweiten Schicht 20 werden die Implantate mit einem dünnen artgleichen Draht (z.B. Ta oder Nb) oder einem Draht aus Ti oder Al elektrisch verbunden und in eine wässrige Elektrolytlösung getaucht und anodisch kontaktiert. Bei der Kontaktierung ist darauf zu achten, dass der Kontaktdruck den Schichtwerkstoff nicht beschädigt und kein elektrischer Kontakt zum Grundwerkstoff stattfindet.

Die wässrige Lösung enthält 100 ml/l Ethylendiamin (99%), 50 g/l Kaliumdihydrogenphosphat, 10 g/l Natriumcarbonat und 25 ml/l 25%-ige wässrige Ammoniaklösung (%-Angaben in Gew.%). Die Zusammensetzung der angegebenen Elektrolytlösung ist so gewählt, dass sich darin enthaltende Elemente oder Verbindungen nach einem anschließend durchzuführenden plasmachemischem Beschichtungsprozess auf der Oberfläche des Implantats wiederfinden. Die Elektrolytlösung befindet sich in einem geeigneten Behälter aus Glas oder Kunststoff. In diesem befindet sich die aus rost- und säurebeständigem Stahl bestehende Gegenelektrode, die hier die Funktion der Kathode übernimmt.

Nach Anlegen einer gepulsten, stetig steigenden anodischen Badspannung mit einer Frequenz von mindestens 1 kHz an das Implantat, die sich durch lange Pulspausen auszeichnet, kommt es sowohl zur Oxidation des Schichtwerkstoffs Ta bzw. Niob als auch zur Bildung von Phosphaten dieser Metalle, so dass die erste Schicht 10 auf der Oberfläche der zweiten Schicht 20 entsteht. Vor dem Beschichtungsprozess muss darauf geachtet werden, dass die Schichtwerkstoffe der zweiten Schicht 20 das Implantat allumfassend bedecken (d.h. keine Fehlstellen aufweist) und der erfindungsgemäße Elektrolyt - in dem die plasmachemischen Effekte stattfinden - keinen Kontakt zum jeweiligen Grundwerkstoff des Körpers 5 erlangt.

Die durch diese plasmachemischen Effekte entstandene Kompositschicht 20 weist eine verfahrensbedingt poröse Oberfläche auf. Die Poren sind zwar statistisch aber doch relativ regelmäßig verteilt und haben einen mittleren Porendurchmesser von ca. 1 bis 3 µm. Die Schicht zeichnet sich durch eine hohe Adhäsion zum jeweiligen Grundwerkstoff (Nitinol, Edelstahl, Magnesium- und/oder Cobalt-Chromlegierungen) aus. Die Dicke und die Oberflächenrauheit der erfindungsgemäßen Oberflächenschicht lässt sich durch die in den nachfolgenden Beispielen beschriebenen Verfahrensparameter beeinflussen. So ist es je nach Anwendungsfall möglich, sowohl eine relativ glatte, glas- oder keramikartige Oberfläche mit überwiegend wurmartigen Poren oder eine relativ raue Oberfläche mit einer mit runden Poren versetzten kraterartigen Oberflächenmorphologie zu erzeugen. Die für die Metalle Tantal und Niob spezifischen Elektrolytendspannungen, die im Bereich zwischen 250 V und 450 V (je nach einzustellender Schichtdicke und Oberflächenrauheit) liegen, sollten nicht überschritten werden. Andernfalls würden die plasmachemischen O-xidationseffekte - in Abhängigkeit von der Dicke des ursprünglich aufgetragenen Schichtwerkstoffs - den jeweiligen Grundwerkstoff erreichen. Die in diesem Fall auftretenden Lichtbogenerscheinungen würden zur Zerstörung des soeben erzeugten Compositschichtverbundes führen. Auch aus diesem Grund sind die plasmachemischen Verfahrensparameter so zu wählen, dass keine vollständige Tiefenoxidation des Werkstoffs der zweiten Schicht 20 stattfindet. Die Oxidation soll höchstens bis in eine Tiefe des Werkstoffs stattfinden, die 1 µm kleiner ist als die ursprüngliche Dicke der zweiten Schicht 20. Somit wird eine verbleibende metallische Schichtwerkstoffdicke der zweiten Schicht 20 von ca. 1 µm sichergestellt, welche im späteren Einsatzfall den Hauptteil der plastischen Verformung (z.B. bei einer Stentdilatation) übernehmen wird. Hierbei ist zu beachten, dass vorzugsweise hochreines Ta bzw. Nb aufgebracht wird, da nur hochreine Elemente das für die vorliegende Anwendung notwendige Plastifizierungsvermögen aufweisen.

Nach Erreichen der für das jeweilige Schichtsystem vor dem Prozessstart fixierten optimalen Endspannung, sinkt die Stromdichte von ca. 1 A/dm² auf ca. 50% des Ausgangswertes. Nach Erreichen dieses Wertes wird der Prozess durch Trennen der Stromzufuhr unterbrochen und das mit der zweiten Schicht 20 und der ersten Schicht 10 versehene Implantat kann dem Elektrolyt entnommen werden. Es erfolgt ein sofortiges mehrstufiges Spülen in ca. 80°C heißem, destilliertem Wasser. Das Spülen dient zur restlosen Entfernung des noch in den Oberflächenrauheiten adsorbierten bzw. chemiesorbierten Restelektrolyts. Schlussendlich erfolgt eine Trocknung in einem Trockenschrank bei ca. 100°C.

Nach diesen Prozessschritten kommen - in Abhängigkeit der Anwendung des Implantats - mannigfaltige Weiterbehandlungen zum Einsatz. Diese können das Tauchen in oder Aufsprühen von polymeren Lösungen zur Herstellung einer auf der ersten Schicht 10 angeordneten vierten Schicht 40 umfassen, die pharmazeutisch aktive Komponenten aufweisen (z.B. für vaskuläre Intervention). Vorzugsweise wird als vierte Schicht 40 eine Parylene enthaltende Schicht aufgebracht (siehe Beispiel 6). Für die Herstellung der vierten Schicht 40 können Lösungen auf der Basis degradierbarer oder nicht degradierbarer Polymere zum Einsatz kommen. Die Auswahl der pharmazeutisch aktiven Substanz(en) erfolgt entsprechend dem jeweiligen Anwendungsfall. Die vierte Schicht kann jedoch auch entfallen.

### Beispiel 3:

Wie Beispiel 2.

Nach dem Trocknen im Trockenschrank erfolgt anstelle des Tauchens in oder Aufsprühen von polymeren Lösungen als vierte Schicht ein Tauchen in wässrige oder nichtwässrige Medien, in denen Bone Morphogenetic Proteins (BMPs) gelöst sind, die durch Anlegen eines Vakuums in die Porenstruktur gesaugt werden, dort nach einem Trocknungsprozess verbleiben und im Falle orthopädischer Implantate das Knochenwachstum positiv beeinflussen.

### Beispiel 4:

Wie Beispiel 2.

Nach dem Trocknen im Trockenschrank erfolgt zur Herstellung einer weiteren alternativen vierten Schicht ein Tauchen in verdünnte Hyaluronsäure, die im Falle von Gelenkimplantaten die Knorpelbildung beschleunigt.

### Beispiel 5:

Wie Beispiel 2.

Nach dem Trocknen im Trockenschrank erfolgt zur Herstellung einer weiteren alternativen vierten Schicht ein Tauchen in magnesiumstearathaltigen Lösungen (Gemisch aus Isopropanol und Magnesiumstearat). Die sich nach dem Trocknen bildende magnesiumstearathaltige Oberfläche minimiert den Reibungskoeffizienten im Katheter und erleichtert damit die Freisetzung z.B. von Stents am Implantationsort.

### Beispiel 6:

Wie Beispiel 2.

Nach dem Trocknen im Trockenschrank erfolgt eine Beschichtung mit Parylene als alternative vierte Schicht mittels Plasmapolymerisation. Die Schichtdicken der Parylene-Schicht (vom Typ C, N oder D) kann dabei zwischen etwa 0,5 µm und etwa 10 µm, vorzugsweise zwischen etwa 0,5 µm und etwa 7 µm betragen. Bei einer solchen Schichtkombination wird die Degradationsstabilität der Endoprothese nochmals wesentlich erhöht sowie der Reibungskoeffizient im Katheter minimiert.

### Beispiel 7:

Wie Beispiel 2.

Anschließend erfolgt zur Herstellung einer weiteren alternativen vierten Schicht eine Beschichtung mit einem nicht resorbierbaren Polymer mit Pulverpartikeln aus Barium (Reinsubstanz), Bismuth oder Wolfram oder deren Karbiden. Mit dieser Beschichtung wird eine weitere Erhöhung der Röntgensichtbarkeit der Endoprothese ermöglicht.

### Beispiel 8:

In den vorstehenden Beispielen kann sich unmittelbar nach der plasmachemischen Beschichtung zur Herstellung der ersten Schicht 10 zusätzlich zu den übrigen Schritten eine Nachbehandlung mit einer wässrigen, stark basischen Natriumhydroxidlösung zur Herstellung der im Wesentlichen in den Porengründen angeordneten dritten Schicht 30 (vgl. Figur 2) anschließen, wobei die zweite Schicht 20, wie in Figur 2 dargestellt, auch entfallen kann. Bei der Nachbehandlung wird der Reaktionsbehälter mit Ultraschall beaufschlagt und gleichzeitig Argon oder Stickstoff mittels einer Fritte eingeblasen. Dadurch wird auch eine chemische Wechselwirkung an den schwer zugänglichen Porengründen 12 ermöglicht. Dieser Prozess lässt eine mehrere 10 nm dicke dritte Schicht 30 in den Porengründen 12 entstehen, welche die Wirkung einer Diffusionsbarriere hat bzw. bei mechanischer Beanspruchung vorteilhaft ist. Diese Barrierewirkung lässt sich noch weiter verstärken, wenn bei einem so behandelten Implantat nach einem zwischengelagerten Trocknungsprozess ein Sauerstoffplasma angewendet wird. Das Sauerstoffplasma bewirkt - je nach Einwirkdauer - eine ganze oder teilweise Umwandlung des in der dritten Schicht 30 enthaltenen Hydroxids des Grundmaterials zu dem Oxid des Grundmaterials (z.B. Umwandlung von Magnesiumhydroxid zu Magnesiumoxid), insbesondere im oberen Bereich der dritten Schicht 30.

In einem weiteren bevorzugten Beispiel erfolgt anschließend in der gleichen Beschichtungsanlage eine Plasmapolymerisation mit einem biokompatiblen Polymer, vorzugsweise mit Parylene N zur Herstellung der vierten Schicht 40. Eine ca. 0,5 bis 7 µm dicke Parylene N-Schicht 40 bedeckt das Implantat umfassend. Durch die hohe Spaltgängigkeit erfolgt auch eine tiefgehende Penetration des Polymers in die Poren bis hin zu den Porengründen.

### Bezugszeichenliste:

- 5: Körper des Implantats
- 10: erste Schicht
- 12: Grund einer Pore in der ersten Schicht 10
- 20: zweite Schicht
- 30: dritte Schicht
- 40: vierte Schicht

## Patentansprüche

1. Verfahren zur Herstellung einer intraluminalen Endoprothese mit einem Körper (5), wobei der Körper (5) metallisches Material aufweist, umfassend die folgenden Schritte:
a) Bereitstellen des Körpers (5) der intraluminalen Endoprothese ,
b) plasmachemische Behandlung mindestens eines Teils der Körperoberfläche in einer wässrigen Lösung mittels Anlegen einer das Plasma generierenden Wechselspannung an den Körper (5) der intraluminalen Endoprothese, die eine Frequenz von mindestens etwa 1 kHz aufweist, zur Erzeugung einer ersten Schicht (10) auf dem behandelten Teil der Körperoberfläche.

2. Verfahren nach Anspruch 1, **dadurch gekennzeichnet, dass** die wässrige Lösung Phosphationen enthält.

3. Verfahren nach Anspruch 1 oder 2, **dadurch gekennzeichnet, dass** der Körper (5) mindestens ein zumindest weitestgehend biodegradierbares metallisches Material aufweist.

4. Verfahren nach Anspruch 3, **dadurch gekennzeichnet, dass** die wässrige Lösung ein Ion oder mehrere Ionen Sr²⁺ enthält, die vorzugsweise in einer Konzentration von jeweils 0,05 Mol/l bis 2,0 Mol/l Sr²⁺ in der wässrigen Lösung enthalten sind.

5. Verfahren nach einem der vorhergehenden Ansprüche, **dadurch gekennzeichnet, dass** vor der plasmachemischen Behandlung auf mindestens ein Teil der Körperoberfläche eine zweite Schicht (20) aufgebracht wird, welche mindestens ein Element der Gruppe bestehend aus Tantal, Niob, Zink, Eisen und Aluminium aufweist, wobei die zweite Schicht (20) mittels einer ionischen Flüssigkeit, Sputtern, Hochratenzerstäuben und/oder Bedampfen aufgebracht wird.

6. Verfahren nach Anspruch 5, **dadurch gekennzeichnet, dass** die ionische Flüssigkeit Pyrrolidinium-Ionen enthält.

7. Verfahren nach einem der vorhergehenden Ansprüche, **dadurch gekennzeichnet, dass** die erste Schicht (10) nach der plasmachemischen Behandlung in einem Lösungsmittel, vorzugsweise mittels destilliertem H₂O gespült und anschließend vorzugsweise bei einer Temperatur von mindestens etwa 80°C, besonders bevorzugt mindestens etwa 100°C, getrocknet wird, wobei die Trocknung vorzugsweise in einem Umluftofen durchgeführt wird.

8. Verfahren nach einem der vorhergehenden Ansprüche, **dadurch gekennzeichnet, dass** die wässrige Lösung zusätzlich ein oder mehrere Ionen ausgewählt aus der Gruppe der Phosphate, Carbonate und Silikate aufweist und/oder dass in der wässrigen Lösung ein Puffer, vorzugsweise Kaliumdihydrogenphosphat und/oder Natriumdihydrogenphosphat enthalten ist.

9. Verfahren nach einem der vorhergehenden Ansprüche, **dadurch gekennzeichnet, dass** die Körperoberfläche vor der plasmachemischen Behandlung oder vor dem Aufbringen der zweiten Schicht (20) elektrochemisch behandelt, vorzugsweise elektrochemisch poliert wird.

10. Verfahren nach einem der vorhergehenden Ansprüche, **dadurch gekennzeichnet, dass** die plasmachemische Behandlung der Körperoberfläche dadurch erfolgt, dass an den Körper (5) eine gepulste Spannung angelegt wird, deren Amplitude im überwiegenden Teil des Behandlungszeitraums mindestens etwa 90 V übersteigt, besonders bevorzugt mindestens etwa 100 V übersteigt und vorzugsweise im Verlauf der Behandlung ansteigt und die Stromdichte bei der plasmachemischen Behandlung mindestens etwa 8 mA/cm², vorzugsweise mindestens etwa 10 mA/cm² beträgt.

11. Verfahren nach einem der vorhergehenden Ansprüche, **dadurch gekennzeichnet, dass** nach der plasmachemischen Behandlung eine Nachbehandlung des Körpers (5) des Implantats in einer wässrigen, stark basischen Natriumhydroxidlösung, vorzugsweise mit einem pH-Wert zwischen etwa 11 und 13,8, zur Erzeugung einer dritten Schicht (30) erfolgt, wobei die Nachbehandlung unter Beaufschlagung von Ultraschall und/oder Einblasen von Argon und/oder Stickstoff durchgeführt wird.

12. Verfahren nach einem der vorhergehenden Ansprüche, **dadurch gekennzeichnet, dass** nach der plasmachemischen Behandlung auf die erste Schicht (10) und/oder auf die dritte Schicht (30) eine vierte, vorzugsweise ein Polymer enthaltende Schicht (40), die besonders bevorzugt mindestens überwiegend aus Parylene besteht, aufgebracht wird.

13. Intraluminale Endoprothese mit einem Körper (5), der mindestens ein metallisches Material aufweist, erhältlich durch ein Verfahren gemäß einem der vorhergehenden Ansprüche, wobei die Dicke der mittels der plasmachemischen Behandlung auf der Körperoberfläche erzeugten ersten Schicht (10) etwa 1 bis etwa 20 µm, vorzugsweise etwa 2 bis etwa 8 µm beträgt und/oder die erste Schicht (10) mindestens ein Phosphat enthält.

14. Intraluminale Endoprothese nach Anspruch 13, **dadurch gekennzeichnet, dass** die zweite Schicht (20) eine Schichtdicke zwischen etwa 0,5 µm und etwa 15 µm aufweist.

15. Intraluminale Endoprothese nach einem der Ansprüche 13 oder 14, **dadurch gekennzeichnet, dass** die erste Schicht (10) Poren aufweist, wobei vorzugsweise als dritte Schicht (30) auf dem Porengrund (12) ein Hydroxid des metallischen Materials des Implantat-Körpers gebildet ist und/oder dass die erste Schicht (10) mindestens eine Verbindung ausgewählt aus der Gruppe enthaltend Phosphate, Hydroxide und Oxide des vorzugsweise biodegradierbaren metallischen Materials oder der vorzugsweise biodegradierbaren metallischen Materialien, Strontiumkarbonat und Strontiumphosphat aufweist.

## Claims

1. A method for producing an intraluminal endoprosthesis having a body (5), wherein the body (5) contains metal material, comprising the following steps:
a) providing the body (5) of the intraluminal endoprosthesis,
b) subjecting at least part of the body surface to a plasma-chemical treatment in an aqueous solution by applying to the body (5) of the intraluminal endoprosthesis an AC voltage, which generates the plasma and has a frequency of at least approximately 1 kHz, in order to produce a first layer (10) on the treated part of the body surface.

2. The method according to Claim 1, **characterised in that** the aqueous solution contains phosphate ions.

3. The method according to Claim 1 or 2, **characterised in that** the body (5) comprises at least one at least largely biodegradable metal material.

4. The method according to Claim 3, **characterised in that** the aqueous solution contains one or more Sr²⁺ ions, which is/are preferably contained in the aqueous solution in a concentration from 0.05 mol/l to 2.0 mol/l Sr²⁺.

5. The method according to one of the preceding claims, **characterised in that**, before the plasma-chemical treatment, a second layer (20) containing at least one element from the group consisting of tantalum, niobium, zinc, iron and aluminium is applied to at least part of the body surface, wherein the second layer (20) is applied by means of an ionic fluid, sputtering, high-rate atomisation and/or vaporisation.

6. The method according to Claim 5, **characterised in that** the ionic fluid contains pyrrolidinium ions.

7. The method according to one of the preceding claims, **characterised in that** the first layer (10), after the plasma-chemical treatment in a solvent, is rinsed preferably by means of distilled H₂O and is then dried preferably at a temperature of at least approximately 80 °C, particularly preferably at least approximately 100 °C, wherein the drying is preferably carried out in a circulating air furnace.

8. The method according to one of the preceding claims, **characterised in that** the aqueous solution additionally contains one or more ions selected from the group comprising phosphates, carbonates and silicates, and/or **in that** a buffer, preferably potassium dihydrogen phosphate and/or sodium dihydrogen phosphate, is contained in the aqueous solution.

9. The method according to one of the preceding claims, **characterised in that** the body surface is electrochemically treated, preferably electrochemically polished, before the plasma-chemical treatment or before the application of the second layer (20).

10. The method according to one of the preceding claims, **characterised in that** the plasma-chemical treatment of the body surface is performed by applying to the body (5) a pulsed voltage, of which the amplitude is in excess of at least approximately 90 V, particularly preferably in excess of at least approximately 100 V, during most of the period of the treatment and preferably increases during the course of the treatment, and the current density during the plasma-chemical treatment is at least approximately 8 mA/cm², preferably at least approximately 10 mA/cm².

11. The method according to one of the preceding claims, **characterised in that**, after the plasma-chemical treatment, an aftertreatment of the body (5) of the implant in an aqueous, strongly alkaline sodium hydroxide solution, preferably with a pH value between approximately 11 and 13.8, is performed in order to produce a third layer (30), wherein the aftertreatment is carried out under the action of ultrasound and/or with injection of argon and/or nitrogen.

12. The method according to one of the preceding claims, **characterised in that**, after the plasma-chemical treatment, a fourth layer (40), preferably containing a polymer and particularly preferably consisting at least predominantly of parylene, is applied to the first layer (10) and/or to the third layer (30).

13. An intraluminal endoprosthesis having a body (5), which contains at least one metal material, obtainable by a method according to one of the preceding claims, wherein the thickness of the first layer (10) produced by means of the plasma-chemical treatment on the body surface is approximately 1 to approximately 20 µm, preferably approximately 2 to approximately 8 µm, and/or the first layer (10) contains at least one phosphate.

14. The intraluminal endoprosthesis according to Claim 13, **characterised in that** the second layer (20) has a layer thickness between approximately 0.5 µm and approximately 15 µm.

15. The intraluminal endoprosthesis according to one of Claims 13 or 14, **characterised in that** the first layer (10) has pores, wherein a hydroxide of the metal material of the implant body is preferably formed as a third layer (30) on the base (12) of the pore, and/or **in that** the first layer (10) contains at least one compound selected from the group containing phosphates, hydroxides and oxides of the preferably biodegradable metal material or of the preferably biodegradable metal materials, strontium carbonate and strontium phosphate.

## Revendications

1. Procédé pour la fabrication d'une endoprothèse intraluminale avec un corps (5), dans lequel le corps (5) comporte un matériau métallique, comprenant les étapes suivantes :
a) mise à disposition du corps (5) de l'endoprothèse intraluminale,
b) traitement chimique au plasma d'au moins une partie de la surface de corps dans une solution aqueuse, en appliquant une tension alternative au corps (5) de l'endoprothèse intraluminale, laquelle présente une fréquence d'au moins environ 1 kHz, pour produire une première couche (10) sur la partie traitée de la surface du corps.

2. Procédé selon la revendication 1, **caractérisé en ce que** la solution aqueuse contient des ions phosphate.

3. Procédé selon la revendication 1 ou 2, **caractérisé en ce que** le corps (5) comporte au moins un matériau métallique au moins majoritairement biodégradable.

4. Procédé selon la revendication 3, **caractérisé en ce que** la solution aqueuse contient un ion ou plusieurs ions Sr²⁺, contenus de préférence selon une concentration respective de 0,05 Mol/l à 2,0 Mol/l de Sr²⁺ dans la solution aqueuse.

5. Procédé selon l'une des revendications précédentes, **caractérisé en ce qu'**une deuxième couche (20) est appliquée sur au moins une partie de la surface de corps avant le traitement chimique au plasma, laquelle comporte au moins un élément parmi le groupe comprenant le tantale, le niobium, le zinc, le fer et l'aluminium, dans lequel la deuxième couche (20) est appliquée au moyen d'un fluide ionique, par pulvérisation, par vaporisation à haut débit et/ou dépôt de vapeur.

6. Procédé selon la revendication 5, **caractérisé en ce que** le liquide ionique contient des ions pyrrolidinium.

7. Procédé selon l'une des revendications précédentes, **caractérisé en ce que** suite au traitement chimique au plasma, la première couche (10) est rincée dans un solvant, de préférence avec du H₂O distillé, puis séchée de préférence à une température d'au moins environ 80°C, et mieux, d'au moins environ 100°C, le séchage étant de préférence effectué dans un four à circulation d'air.

8. Procédé selon l'une des revendications précédentes, **caractérisé en ce que** la solution aqueuse comporte en outre un ou plusieurs ions choisis parmi le groupe des phosphates, des carbonates et des silicates et/ou **en ce que** la solution aqueuse contient un tampon, de préférence du phosphate d'hydrogène de potassium et/ou du phosphate de dihydrogène de potassium.

9. Procédé selon l'une des revendications précédentes, **caractérisé en ce que** la surface de corps est traitée électrochimiquement, de préférence polie électrochimiquement, avant le traitement chimique au plasma ou avant l'application de la deuxième couche (20).

10. Procédé selon l'une des revendications précédentes, **caractérisé en ce que** le traitement chimique au plasma de la surface de corps est réalisé en appliquant une tension pulsée au corps (5), dont l'amplitude dépasse au moins environ 90 V sur la majeure partie de la durée de traitement, dépassant de façon particulièrement préférentielle au moins environ 100 V, et augmentant de préférence au cours du traitement, et **en ce que** la densité de courant est d'au moins environ 8 mA/cm², de préférence d'au moins environ 10 mA/cm² pendant le traitement chimique au plasma.

11. Procédé selon l'une des revendications précédentes, **caractérisé en ce que** suite au traitement chimique au plasma, un traitement ultérieur du corps (5) de l'implant est réalisé dans une solution aqueuse d'hydroxyde de natrium fortement basique, de préférence avec une valeur de pH entre environ 11 et 13,8, pour la production d'une troisième couche (30), le traitement ultérieur étant effectué en appliquant un ultrason et/ou en soufflant de l'argon et/ou de l'azote.

12. Procédé selon l'une des revendications précédentes, **caractérisé en ce que** suite au traitement chimique au plasma, une quatrième couche (40) contenant de préférence un polymère et constituée de façon particulièrement préférentielle au moins majoritairement de parylène est appliquée sur la première couche (10) et/ou sur la troisième couche (30).

13. Endoprothèse intraluminale avec un corps (5) comportant au moins un matériau métallique, lequel peut être obtenu à l'aide d'un procédé selon l'une des revendications précédentes, dans laquelle l'épaisseur de la première couche (10) produite au moyen du traitement chimique au plasma sur la surface de corps mesure entre environ 1 et environ 20 µm, de préférence entre environ 2 et environ 8 µm, et/ou la première couche (10) contient au moins un phosphate.

14. Endoprothèse intraluminale selon la revendication 13, **caractérisée en ce que** la deuxième couche (20) présente une épaisseur de couche entre environ 0,5 µm et environ 15 5 µm.

15. Endoprothèse intraluminale selon l'une des revendications 13 ou 14, **caractérisée en ce que** la première couche (10) comporte des pores, un hydroxyde du matériau métallique du corps d'implant étant de préférence formé en tant que troisième couche (30) sur la base poreuse (12), et/ou **en ce que** la première couche (10) comporte au moins une combinaison sélectionnée parmi le groupe contenant du phosphate, de l'hydroxyde et de l'oxyde du matériau métallique de préférence biodégradable ou des matériaux métalliques de préférence biodégradables, du carbone de strontium et du phosphate de strontium
